# EUROPEAN PATENT APPLICATION

(11) **EP 0 574 148 A1**
(43) Date of publication of application: **15.12.1993**
(21) Application number: 93303927.3
(22) Date of filing: 20.05.1993
(51) Int. Cl.: C09B 57/00, C07D 493/04

(54) **Polycyclic dyes**

(30) Priority: 11.06.1992 GB 9212411
(71) Applicant: ZENECA LIMITED, London W1Y 6LN (GB)
(72) Inventor: Patel, Prakash, Edgerton, Huddersfield HD3 3AX (GB)
(74) Representative: Giles, David Eric

(57) **Abstract**

A polycyclic dye of the Formula (1): 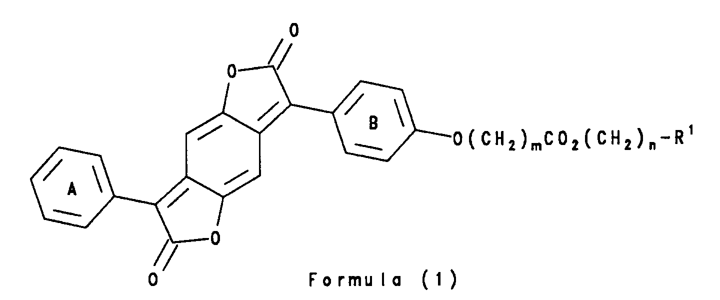
wherein:
- Ring A: is unsubstituted or is substituted by from 1 to 3 groups;
- Ring B: is unsubstituted apart from the O(CH₂)ₘCO₂(CH₂)ₙ-R¹ group or is substituted by from 1 to 4 further groups;
- R¹: is -H, alkoxy or a 5, 6 or 7-membered saturated or unsaturated heterocyclic residue;
- m: is an integer from 1 to 6; and
- n: is from 0 to 6.

These polycyclic dyes are useful for the coloration of synthetic textiles, particularly polyester textile materials to which they impart a bluish-red shade.

## Description

This specification describes an invention which relates to polycyclic dyes.

According to the present invention there is provided a polycyclic dye of the Formula (1): 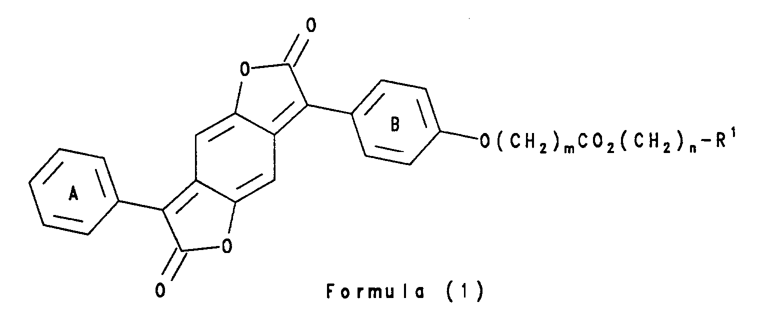
wherein:
- Ring A: is unsubstituted or is substituted by from 1 to 3 groups;
- Ring B: is unsubstituted apart from the O(CH₂)ₘCO₂(CH₂)ₙ-R¹ group or is substituted by from 1 to 4 further groups;
- R¹: is -H, alkoxy or a 5, 6 or 7-membered saturated or unsaturated heterocyclic residue;
- m: is an integer from 1 to 6; and
- n: is from 0 to 6.

A preferred sub group of dyes of Formula (1) are those in which R¹ is a 5, 6 or 7-membered saturated or unsaturated heterocyclic residue, n is an integer from 1 to 6 and Ring A, Ring B and m are as hereinbefore defined.

A further preferred sub group of polycyclic dye of Formula (1) is preferably of the Formula (2): 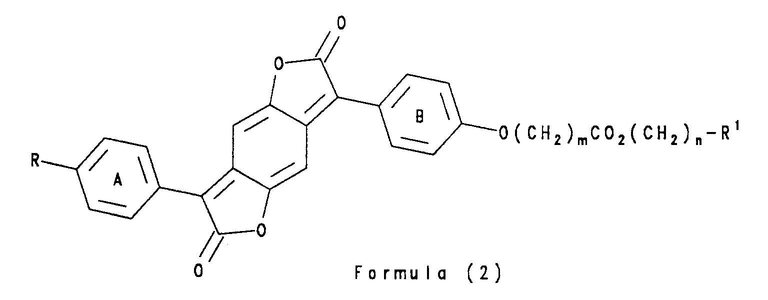
wherein:
- R: is -H, -OH, halogen, alkyl, alkenyl, alkoxy, aryl, -O(CH₂)ₘR⁴ in which R⁴ is a 5, 6 or 7-membered saturated or unsaturated heterocyclic residue or -NR²R³ in which R² and R³ are each independently -H, alkyl or alkenyl;
- each m: independently is an integer from 1 to 6;
- n: is from 0 to 6; and
- Ring A, Ring B and R¹: are as hereinbefore defined.

The alkyl group represented by R, R² and R³ may be a straight or branched chain alkyl group and is preferably C₁₋₆-alkyl and more preferably C₁₋₄-alkyl. The alkoxy group represented by R may be a straight or branched chain alkoxy group and is preferably C₁₋₆-alkoxy and more preferably C₁₋₄-alkoxy. The aryl group represented by R is preferably phenyl or naphthyl. The alkenyl group represented by R, R² and R³ is preferably C₂₋₁₀-alkenyl and more preferably C₃₋₄-alkenyl. The halogen represented by R is preferably -F, -Cl or -Br.

The alkyl, alkenyl, alkoxy and aryl groups represented by R, the alkyl or alkenyl groups represented by R² and R³ and the heterocyclic residues represented by R¹ may be substituted by at least one group selected from C₁₋₆-alkyl, C₁₋₆-alkoxy, hydroxy, cyano, halogen, such as -Cl and -F, nitro, C₁₋₄-alkoxy-C₁₋₄-alkoxy, C₁₋₄-alkylcarbonyl, C₁₋₄-alkoxycarbonyl, C₁₋₄-alkoxy-C₁₋₄-alkoxy carbonyl, C₁₋₄-alkylcarbonyloxy, C₁₋₄-alkoxycarbonyloxy, C₁₋₄-alkoxy-C₁₋₄-alkoxycarbonyloxy, phenyl, amino, C₁₋₄-alkylamino and di(C₁₋₄-alkyl)amino.

Any of the substituents described above for R, R¹, R² and R³ are suitable substituents for Ring A and Ring B. Where Ring A is substituted by one further group in addition to the group R it is preferred that this group is in the 3-position and where substituted by two further groups it is preferred that these groups are in the 3- and 5-positions. Where Ring B is substituted by one or two further groups it is preferred that these groups are in the 3- or in the 3- and 5-positions of Ring B.

The heterocyclic residue represented by R¹ or R⁴ is preferably a 5- or 6-membered saturated or unsaturated heterocyclic residue and is preferably pyranyl, tetrahydropyranyl, furyl, tetrahydrofuryl, thienyl, tetrahydrothienyl, pyrrolyl, pyrrolidyl, pyridyl, piperidyl, thiazolyl, isothiazolyl and morpholinyl, more preferably tetrahydropyranyl and tetrahydrofuryl and especially tetrahydropyran-2-yl and tetrahydrofur-2-yl.

The compounds of Formula (1) may be prepared by esterification of the corresponding carboxylic acid with an alcohol of Formula HO(CH₂)ₙ-R¹ in which n and R¹ are as hereinbefore defined. The corresponding carboxylic acids referred to above may be prepared by the method described in European patent 0146269A.

The compounds of the present invention give bluish-red shades when applied to synthetic fibres such as polyester and exhibit excellent fastness properties.

The invention is further illustrated by the following examples.

### Example 1

### Preparation of 3-(4-(n-propoxy)phenyl)-7-(4-(tetrahydropyran-2-yl methoxycarbonylmethoxy)phenyl)-2,6-dioxo-2,6-dihydrobenzo[1:2-b, 4:5-b']difuran

i) A mixture of hydroquinone (15g), 4-n-propoxymandelic acid (21g), acetic acid (95cm³) and sulphuric acid (5cm³) was stirred at ambient temperature for 14 hours before filtering off a solid. The solid was washed with water and dichloromethane and dried to give 5-hydroxy-2-oxo- 3-(4-n-propoxy)phenyl-2,3-dihydrobenzofuran (19g, 67%).
ii) A mixture of 5-hydroxy-2-oxo-3-(4-n-propoxy)phenyl-2,3-dihydrobenzofuran (14g), 4-carboxymethoxymandelic acid (14g), acetic acid (75cm³) and sulphuric acid (7.5cm³) was heated at 110°C for 18 hours before cooling. Ammonium persulphate (10g) was added and the mixture heated at 110°C for 1 hour. The reaction mixture was cooled and poured into a mixture of ice and water, the precipitated solid was filtered off, washed with water and methanol and dried to give 3-(4-n-propoxy)phenyl-7-(4-(carboxymethoxy)phenyl)-2,6-dioxo-2,6-dihydrobenzo[1:2-b, 4:5-b']difuran (10.5, 44%).
iii) A mixture of 3-(4-(n-propoxy)phenyl)-7-(4-(carboxymethoxy) phenyl)-2,6-dioxo-2,6-dihydrobenzo[1:2-b, 4:5-b']difuran (3g), tetrahydropyran-2-ylmethanol (30cm³) and sulphuric acid (0.5cm³) were heated at 190°C for 1 hour. The reaction mixture was cooled and the precipitated solid was filtered off, washed with water and with methanol and dried. The dried solid was recrystallised from toluene to give 3-((4-n-propoxy)phenyl)-7-(4-(tetrahydropyran-2-yl-methoxycarbonyl methoxy)phenyl)2,6-dioxo-2,6-dihydrobenzo[1:2-b, 4:5-b'] difuran (1.6g, 47%). Lambda ₘₐₓ in dichloromethane = 521nm.

### Example 2

### Preparation of 3-(4-(n-propoxy)phenyl)-7-(4-(tetrahydrofuran-2-yl methoxycarbonylmethoxy)phenyl)-2,6-dioxo-2,6-dihydrobenzo[1:2-b, 4:5-b']difuran

The procedure of Example 1iii) was followed except that tetrahydrofurfuryl alcohol was used in place of the tetrahydropyran-2-yl methanol to give 3-(4-(n-propoxy)phenyl)-7-(4-(tetrahydrofuran-2-yl methoxcarbonylmethoxy)phenyl)-2,6-dioxo-2,6-dihydrobenzo[1:2-b, 4:5-b']difuran (1.0g, 29%). Lambda ₘₐₓ in dichloromethane = 520nm.

### Example 3

### Preparation of 3-(4-(tetrahydropyran-2-ylmethoxy)phenyl)-7-(4-(carboxy methoxy)phenyl)-2,6-dioxo-2,6-dihydrobenzo[1:2-b, 4:5-b']difuran

i) A mixture of sodium hydroxymandelate (25g, 70%) and sodium hydroxide (4.8g) was dissolved in water (150cm³) and tetrahydropyran-2-ylmethylbromide (20g) was added before stirring at ambient temperature for 16 hours. The reaction mixture was acidified with hydrochloric acid (32%) and extracted with diethyl ether. The diethyl ether was dried over magnesium sulphate, decanted and evaporated to leave 4-(tetra hydropyran-2-ylmethoxy)mandelic acid (14g, 53%) as a brown oil.
ii) A mixture of sulphuric acid (60cm³, 73%), hydroquinone (33g) and 4-carboxymethoxymandelic acid (63.6g, 74.6%) was stirred and heated to 50°C. After stirring for 4 hours at 50°C the reaction mixture was cooled and poured into ice/water (1800g). The precipitated solid was filtered off, washed with water until acid free and dried at 45°C to give 5-hydroxy-2-oxo-3-(4-(carboxymethoxy)phenyl)-2,3-dihydrobenzofuran (47g, 75%).
iii) A mixture of 5-hydroxy-2-oxo-3-(4-(carboxymethoxy)phenyl)-2,3-dihydrobensofuran (7g), 4-(tetrahydropyran-2-ylmethoxy)mandelic acid (14g), acetic acid (2.5cm³) and sulphuric acid (47.5cm³) was stirred and heated at 110°C for 2 hours. The reaction mixture was cooled to 70°C, ammonium persulphate (10g) was added and the reaction mixture was heated at 120°C for ½ hour. The reaction mixture was cooled, ice/water added and the precipitated solid was filtered off, washed with water and methanol and dried to give
   3-(4-(tetrahydropyran-2-ylmethoxy)phenyl)-7-(4-(carboxymethoxy)phenyl)-2,6-dioxo-2,6-dihydrobenzo[1:2-b, 4:5-b']difuran (10g, 81%) as a red solid.

### Example 4

### Preparation of 3-(4-(tetrahydropyran-2-ylmethoxy)phenyl)-7-(4-(2-ethoxy ethoxycarbonylmethoxy)phenyl)-2,6-dioxo-2,6-dihydrobenzo[1:2-b, 4:5-b']difuran

A mixture of 3-(4-(tetrahydropyran-2-ylmethoxy)phenyl)-7-(4-(carboxymethoxy)phenyl)-2,6-dioxo-2,6-dihydrobenzo[1:2-b, 4:5-b'] difuran (5g), 2-ethoxyethanol (20cm³) and sulphuric acid (0.25cm³) was stirred and heated at 160°C for ½ hour. The reaction mixture was cooled, methanol added and the precipitated solid was filtered off and dried. The solid was purified by elution from silica using dichloromethane with up to 5% ethylacetate dichloromethane to give 3-(4-(tetrahydropyran-2-ylmethoxy)phenyl)-7-(4-(2-ethoxyethoxycarbonyl methoxy)phenyl)-2,6-dioxo-2,6dihydrobenzo[1:2-b, 4:5-b']difuran (0.8g, 14%) as a dark bluish red solid. Lambda ₘₐₓ in dichloromethane = 518nm.

### Example 5

### Preparation of 3-(4-(tetrahydropyran-2-ylmethoxy)phenyl)-7-(4-(tetrahydrofuran-2-ylmethoxycarbonylmethoxy)phenyl)-2,6-dioxo-2,6-dihydrobenzo[1:2-b, 4:5-b']difuran

A mixture of 3-(4-(tetrahydropyran-2-ylmethoxy)phenyl)-7-(4-carboxylmethoxy)phenyl)-2,6-dioxo-2,6-dihydrobenzo[1:2-b, 4:5-b'] difuran, tetrahydrofurfurylalcohol (20cm³) and sulphuric acid (0.25cm³) was stirred and heated at 160°C for ½ hour. The reaction mixture was cooled, methanol added and the precipitated solid was filtered off and dried. The solid was purified by elution from silica using dichloromethane with up to 5% ethylacetate dichloromethane to give 3-(4-(tetrahydropyran-2-ylmethoxy)phenyl)-7-(4-tetrahydrofuran-2-yl methoxycarbonylmethoxy)phenyl)-2,6-dioxo-2,6-dihydrobenzo[1:2-b, 4:5-b']difuran (0.7g, 12%) as a dark bluish red solid. Lambda ₘₐₓ in dichloromethane = 518nm.

### Example 6

### Preparation of 3-(4-(tetrahydrofuran-2-ylmethoxy)phenyl)-7-(4-(carboxy methoxy)phenyl)-2,6-dioxo-2,6-dihydrobenzo[1:2-b, 4:5-b']difuran

i) A mixture of sodium hydroxymandelate (75g, 70%) and sodium hydroxide (14g) was dissolved in water (200cm³) and tetrahydrofurfuryl bromide (60g) was added before stirring under reflux for 6 hours. The reaction mixture was cooled and acidified with hydrochloric acid (32%) and the precipitated solid was filtered off to give 4-(tetrahydrofuran -2-ylmethoxy)mandelic acid (22g, 35%) as a white solid.
ii) A mixture of hydroquinone (11.5g), 4-(tetrahydrofuran-2-yl methoxy)mandelic acid (22g), acetic acid (2.5cm³) and sulphuric acid (47.5cm³) were stirred at ambient temperature for 14 hours. The reaction mixture was poured into water to give a sticky solid, titrituration with methanol gave a pink solid which was filtered off to give 5-hydroxy-2-oxo-3-(4-(tetrahydrofuran-2-ylmethoxy)phenyl)-2,3-dihydrobenzofuran (1.7g, 6%).
iii) A mixture of 5-hydroxy-2-oxo-3-(4-(tetrahydrofuran-2-yl methoxy)phenyl)-2,3-dihydrobenzofuran (1.5g), 4-(carboxymethoxy)mandelic acid (1.25g, 75%), acetic acid (1.2cm³) and sulphuric acid (23.8cm³) was stirred and heated at 110°C for 2 hours. The reaction mixture was cooled to 70°C, ammonium persulphate (1g) was added and the reaction mixture was heated at 120°C for ½ hour. The reaction mixture was cooled, ice/water added and the precipitated solid was filtered off, washed with water and methanol and dried to give 3-(4-(tetrahydrofuran-2-ylmethoxy)phenyl)-7-(4-(carboxymethoxy)phenyl)-2,6-dioxo-2,6-dihydro benzo[1:2-b, 4:5-b']difuran (1.6g, 68%) as a red solid.

### Example 7

### Preparation of 3-(4-(tetrahydrofuran-2-ylmethoxy)phenyl)-7-(4-(2-ethoxy ethoxycarbonylmethoxy)phenyl)-2,6-dioxo-2,6-dihydrobenzo[1:2-b, 4:5-b'] difuran

A mixture of 3-(4-(tetrahydrofuran-2-ylmethoxy)phenyl-7-(4-carboxymethoxy)phenyl)-2,6-dihydrobenzo[1:2-b, 4:5-b']difuran (1.6g), 2-ethoxyethanol (20cm³) and sulphuric acid (0.25cm³) was refluxed for ½ hour. The reaction mixture was cooled, methanol added and the precipitated solid was filtered off and dried. The solid was purified by elution from silica using dichloromethane with up to 5% ethylacetate as eluent, the purified solid was recrystallised from dichloromethane to give 3-(4-(tetrahydrofuran-2-ylmethoxy)phenyl-7-(4-(2-ethoxyethoxy carbonylmethoxy)phenyl)-2,6-dioxo-2,6-dihydrobenzo[1:2-b, 4:5-b']difuran (0.7g, 38%) as a dark bluish red solid. Lambda ₘₐₓ in dichloromethane = 516nm.

## Claims

1. A polycyclic dye of the Formula (1): 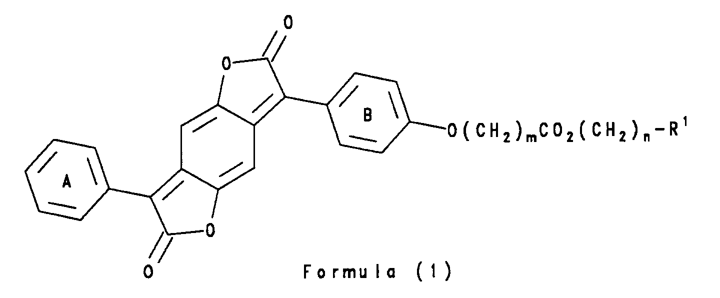 wherein:
Ring A is unsubstituted or is substituted by from 1 to 3 groups;
Ring B is unsubstituted apart from the O(CH₂)ₘCO₂(CH₂)ₙ-R¹ group or is substituted by from 1 to 4 further groups;
R¹ is -H, alkoxy or a 5, 6 or 7-membered saturated or unsaturated heterocyclic residue;
m is an integer from 1 to 6; and
n is from 0 to 6.

2. A polycyclic dye according to Claim 1 wherein R¹ is a 5, 6 or 7-membered saturated or unsaturated heterocyclic residue and n is an integer from 1 to 6.

3. A polycyclic dye according to Claim 1 or Claim 2 wherein R¹ is a 5- or 6-membered saturated or unsaturated heterocyclic residue.

4. A polycyclic dye according to any one of Claims 1 to 3 wherein R¹ is pyranyl, tetrahydropyranyl, furyl, tetrahydrofuryl, thienyl, tetrahydrothienyl, pyrrolyl, pyrrolidyl, pyridyl, piperidyl, thiazolyl, isothiazolyl and morpholinyl.

5. A polycyclic dye according to any one of Claims 1 to 4 wherein R¹ is tetrahydropyranyl or tetrahydrofuranyl.

6. A polycyclic dye of the Formula (2): 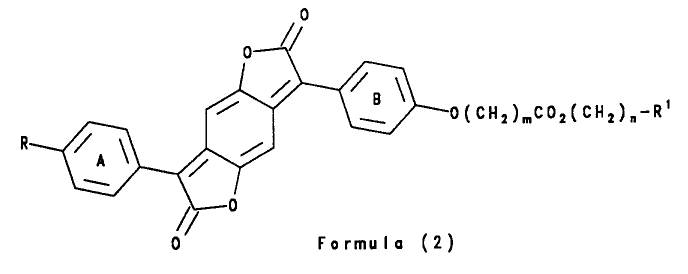 wherein:
R is -H, -OH, halogen, alkyl, alkenyl, alkoxy, aryl, -O(CH₂)ₘR⁴ in which R⁴ is a 5, 6 or 7-membered saturated or unsaturated heterocyclic residue, -NR²R³ in which R² and R³ are each independently -H, alkyl or alkenyl;
Ring A is unsubstituted or is substituted by from 1 to 3 groups;
Ring B is unsubstituted apart from the O(CH₂)ₘCO₂(CH₂)ₙ-R¹ group or is substituted by from 1 to 4 further groups;
R¹ is -H, alkoxy or a 5, 6 or 7-membered saturated or unsaturated heterocyclic residue;
m is an integer for 1 to 6; and
n is from 0 to 6.

7. 3-(4-(n-Propoxy)phenyl)-7-(4-(tetrahydropyran-2-ylmethoxy carbonylmethoxy)phenyl)-2,6-dioxo-2,6-dihydrobenzo[1:2-b, 4:5-b']difuran;
3-(4-(n-propoxy)phenyl)-7-(4-(tetrahydrofuran-2-ylmethoxycarbonyl methoxy)phenyl)-2,6-dioxo-2,6-dihydrobenzo[1:2-b, 4:5-b']difuran; 3-(4-(tetrahydropyran-2-ylmethoxy)phenyl)-7-(4-carboxymethoxy)phenyl-2,6-dioxo-2,6-dihydrobenzo[1:2-b, 4:5-b']difuran;
3-(4-(tetrahydropyran-2-ylmethoxy)phenyl)-7-(4-(2-ethoxyethoxycarbonyl methoxy)phenyl)-2,6-dioxo-2,6-dihydrobenzo[1:2-b, 4:5-b']difuran; 3-(4-(tetrahydropyran-2-ylmethoxy)phenyl)-7-(4-(tetrahydrofuran-2-yl methoxycarbonylmethoxy)phenyl)-2,6-dioxo-2,6-dihydrobenzo[1:2-b, 4:5-b']difuran;
3-(4-tetrahydrofuran-2-ylmethoxy)phenyl-7-(4-(carboxymethoxy)phenyl)-2,6-dioxo-2,6-dihydrobenzo[1:2-b, 4:5-b']difuran;
3-(4-(tetrahydrofuran-2-ylmethoxy)phenyl)-7-(4-(2-ethoxyethoxycarbonyl methoxy)phenyl)-2,6-dioxo-2,6=dihydrobenzo[1:2-b, 4:5-b']difuran.
